(19) Europäisches Patentamt
European Patent Office
Office européen des brevets

(11) Veröffentlichungsnummer: **0 142 075**
**A2**

(12) # EUROPÄISCHE PATENTANMELDUNG

(21) Anmeldenummer: 84112738.4

(22) Anmeldetag: 23.10.84

(51) Int. Cl.⁴: C 07 D 473/00
A 61 K 31/52

(30) Priorität: 05.11.83 DE 3340076

(43) Veröffentlichungstag der Anmeldung: 22.05.85 Patentblatt 85/21

(84) Benannte Vertragsstaaten: AT BE CH DE FR GB IT LI LU NL SE

(71) Anmelder: Dr. Karl Thomae GmbH
Postfach 1755
D-7950 Biberach (Riss)(DE)

(72) Erfinder: Hauel, Norbert, Dr. Dipl.-Chem.
Händelstrasse 12
D-7950 Biberach 1(DE)

(72) Erfinder: Austel, Volkhard, Dr. Dipl.-Chem.
Kapellenweg 7
D-7950 Biberach 1(DE)

(72) Erfinder: Heider, Joachim, Dr. Dipl.-Chem.
Am Hang 3
D-7951 Warthausen 1(DE)

(72) Erfinder: Reiffen, Manfred, Dr. Dipl.-Chem.
Matthias-Erzberger-Strasse 40
D-7950 Biberach 1(DE)

(72) Erfinder: van Meel, Jacobus Constantinus Antonius, Dr
Amriswilstrasse 7
D-7950 Biberach 1(DE)

(72) Erfinder: Diederen, Willi, Dr.
Haldenstrasse 1a
D-7950 Biberach 1(DE)

(54) **Neue Purinderivate, ihre Herstellung und diese Verbindungen enthaltende Arzneimittel.**

(57) Die Erfindung betrifft neue Purinderivate der allgemeinen Formel

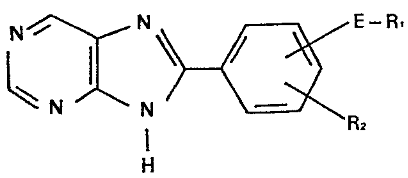

(I)

in der
E ein Sauerstoff- oder Schwefelatom, eine Imino-, Sulfinyl- oder Sulfonylgruppe,

$R_1$ eine Alkylgruppe mit 1 bis 3 Kohlenstoffatomen, welche durch eine Cyan-, Carboxy- oder Alkoxycarbonylgruppe mit insgesamt 2 bis 6 Kohlenstoffatomen substituiert ist, eine Alkenyl- oder Alkinylgruppe mit jeweils 3 bis 5 Kohlenstoffatomen und

$R_2$ eine Alkoxy-, Cyanoalkoxy-, Alkylamino- oder Dialkylaminogruppe, wobei der Alkylteil jeweils 1 bis 3 Kohlenstoffatome enthalten kann, eine Alkenyloxy- oder Alkinyloxygruppe mit jeweils 3 bis 5 Kohlenstoffatomen bedeuten, deren Tautomere und deren Säureadditionssalze, diese Verbindungen enthaltende Arzneimittel und Verfahren zu ihrer Herstellung.

Die neuen Verbindungen weisen wertvolle pharmakologische Eigenschaften auf, insbesondere positiv inotrope Wirkungen und/oder eine Wirkung auf den Blutdruck, und lassen sich nach für analoge Verbindungen bekannten Verfahren herstellen.

EP 0 142 075 A2

Croydon Printing Company Ltd.

DR. KARL THOMAE GMBH
D-7950 Biberach 1

Case 5/886
Dr.Fl./Kp.



# Neue Purinderivate, ihre Herstellung und diese Verbindungen enthaltende Arzneimittel

Gegenstand der vorliegenden Erfindung sind neue Purinderivate der allgemeinen Formel

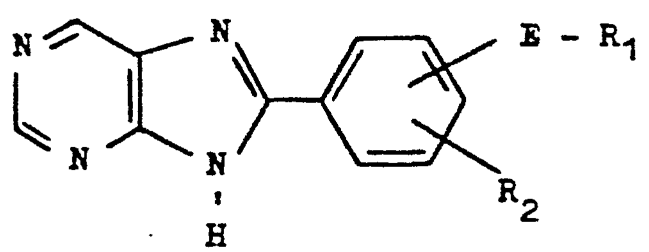

(I)

deren Tautomere und deren Säureadditionssalze, insbesondere deren physiologisch verträgliche Säureadditionssalze mit anorganischen oder organischen Säuren, welche wertvolle pharmakologische Eigenschaften aufweisen, insbesondere eine Wirkung auf den Blutdruck und auf die Kontraktilität des Herzmuskels, diese Verbindungen enthaltende Arzneimittel und Verfahren zu ihrer Herstellung.

In der obigen allgemeinen Formel I bedeutet

E ein Sauerstoff- oder Schwefelatom, eine Imino-, Sulfinyl- oder Sulfonylgruppe,

$R_1$ eine Alkylgruppe mit 1 bis 3 Kohlenstoffatomen, welche durch eine Cyan-, Carboxy- oder Alkoxycarbonylgruppe mit insgesamt 2 bis 6 Kohlenstoffatomen substituiert ist, eine Alkenyl- oder Alkinylgruppe mit jeweils 3 bis 5 Kohlenstoffatomen und

$R_2$ eine Alkoxy-, Cyanoalkoxy-, Alkylamino- oder Dialkylaminogruppe, wobei der Alkylteil jeweils 1 bis 3 Kohlenstoffatome enthalten kann, eine Alkenyloxy- oder Alkinyloxygruppe mit jeweils 3 bis 5 Kohlenstoffatomen.

Für die bei der Definition der Reste eingangs erwähnten Bedeutungen kommt beispielsweise

für $R_1$ die Bedeutung der Cyanomethyl-, 1-Cyanoethyl-, 2-Cyanoethyl-, 3-Cyanopropyl-, Carboxymethyl-, 2-Carboxyethyl-, 3-Carboxypropyl-, Methoxycarbonylmethyl-, Ethoxycarbonylmethyl-, n-Propoxycarbonylmethyl-, Isopropoxycarbonylmethyl-, n-Butoxycarbonylmethyl-, n-Pentoxycarbonylmethyl-, 2-Ethoxycarbonylethyl-, 3-Ethoxycarbonylpropyl-, Allyl-, Buten-2-yl-, Penten-2-yl-, Propargyl-, Butin-2-yl-oder Pentin-2-yl-gruppe und

für $R_2$ die der Methoxy-, Ethoxy-, Propoxy-, Isopropoxy-, Allyloxy-, Buten-2-yloxy-, Penten-2-yloxy-, Propargyloxy-, Butin-2-yloxy-, Pentin-2-yloxy-, Cyanomethoxy-, 1-Cyanoethoxy-, 2-Cyanoethoxy-, 3-Cyano-propoxy-, Methylamino-, Ethylamino-, n-Propylamino-, Dimethylamino-, Diethylamino-, Din-propylamino-, Diisopropylamino-, Methyl-ethylamino- oder Ethyl-isopropylaminogruppe in Betracht.

Bevorzugte Verbindungen der obigen allgemeinen Formel I sind diejenigen, in denen

E ein Sauerstoffatom, eine Imino-, Sulfenyl-, Sulfinyl- oder Sulfonylgruppe,

$R_1$ eine Allyl-, Propargyl-, Cyanomethyl- oder Alkoxycarbonylmethylgruppe, in der der Alkoxyteil 1 bis 3 Kohlenstoffatome enthalten kann, und

$R_2$ eine Methoxy-, Ethoxy-, Allyloxy-, Propargyloxy-, Cyanomethoxy-, oder Dimethylaminogruppe bedeuten,

insbesondere diejenigen Verbindungen, in denen der Rest $R_1$-E- in 4-Stellung und der Rest $R_2$ in 2-Stellung des Phenylkerns steht, deren Tautomere und deren Säureadditionssalze, insbesondere deren physiologisch verträgliche Säureadditionssalze mit anorganischen oder organischen Säuren.

Erfindungsgemäß erhält man die neuen Verbindungen nach folgenden Verfahren:

a) Cyclisierung einer gegebenenfalls im Reaktionsgemisch hergestellten Verbindung der allgemeinen Formel

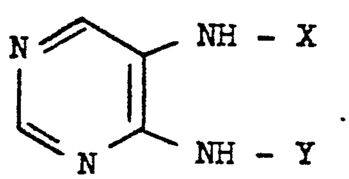

(II)

in der
einer der Reste X oder Y ein Wasserstoffatom und der andere der beiden Reste X und Y oder beide Reste X und Y eine Gruppe der Formel

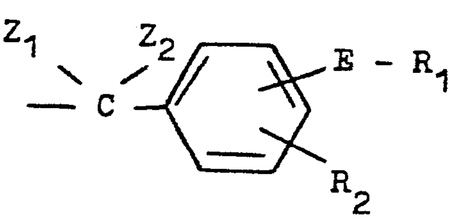

darstellen, in der

$R_1$, $R_2$ und E wie eingangs definiert sind,
$Z_1$ und $Z_2$, die gleich oder verschieden sein können,

gegebenenfalls substituierte Aminogruppen oder gegebenenfalls durch niedere Alkylgruppen substituierte Hydroxy- oder Mercaptogruppen oder

$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittels, z.B. in dem entsprechenden Nitril, Anhydrid, Säurehalogenid, Ester, Amid oder Methojodid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Salzsäure, Phosphorsäure, Polyphosphorsäure, Essigsäureanhydrid oder gegebenenfalls auch in Gegenwart einer Base wie Kaliumäthylat oder Kalium-tert.butylat durchgeführt. Die Cyclisierung kann jedoch auch ohne Lösungsmittel und/oder Kondensationsmittel durchgeführt werden.

b) Zur Herstellung von Verbindungen der allgemeinen Formel I, in der E eine Sulfinyl- oder Sulfonylgruppe darstellt:

Oxidation einer Verbindung der allgemeinen Formel

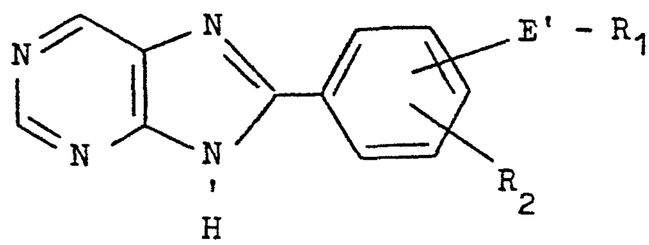

,(III)

in der

$R_1$ und $R_2$ wie eingangs definiert sind und

E' eine Sulfenyl- oder Sulfinylgruppe darstellt.

Die Oxidation wird vorzugsweise in einem Lösungsmittel oder Lösungsmittelgemisch, z.B. in Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer Sulfinylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wässrigem Methanol oder Äthanol bei -15 bis 25°C, mit Brom in Eisessig oder wässriger Essigsäure, mit N-Brom-succinimid in Äthanol, mit tert.Butyl-hyprochlorit in Methanol bei -80 bis -30°C, mit Jodbenzodichlorid in wässrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioäther-Chlor-Komplex wird zweckmäßigerweise mit wäßrigem Äthanol hydrolysiert.

Zur Herstellung einer Sulfonylverbindung der allgemeinen Formel I wird die Oxidation zweckmäßigerweise ausgehend von einer entsprechenden Sulfinylverbindung mit einem bzw. ausgehend von einer entsprechenden Sulfenylverbindung mit zwei oder mehr Äquivalenten des verwendeten Oxidationsmittels durchgeführt, z.B. mit Wasserstoffperoxid in Eisessig, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

c) Zur Herstellung von Verbindungen der allgemeinen Formel I, in der E eine Iminogruppe, ein Sauerstoff- oder ein Schwefelatom darstellt:
Umsetzung einer Verbindung der allgemeinen Formel

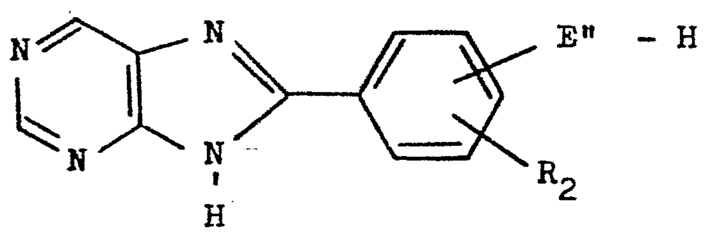

(IV)

in der
$R_2$ wie eingangs definiert ist und
E" eine Iminogruppe, ein Sauerstoff- oder Schwefelatom darstellt, mit einer Verbindung der allgemeinen Formel

$$R_1 - V \quad ,(V)$$

in der
$R_1$ wie eingangs definiert ist und
V eine nukleophile Austrittsgruppe wie ein Halogenatom, z.B. ein Chlor-, Brom- oder Jodatom, darstellt.

Die Umsetzung wird zweckmäßigerweise in einem Lösungsmittel oder Lösungsmittelgemisch wie Methylenchlorid, Ether, Tetrahydrofuran, Dioxan oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

d) Zur Herstellung von Verbindungen der allgemeinen Formel I, in der E ein Sauerstoffatom oder eine Sulfenylgruppe darstellt:

Umsetzung einer Verbindung der allgemeinen Formel

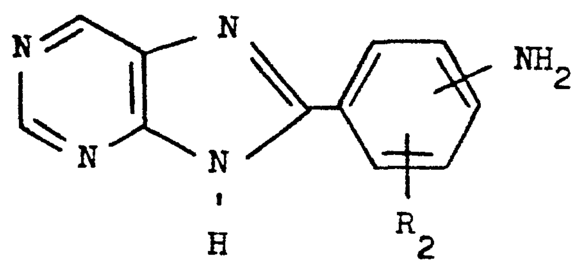

,(VI)

in der

$R_2$ wie eingangs definiert ist, mit einem Nitrit und anschließende Umsetzung des so erhaltenen Diazoniumsalzes mit einer Verbindung der allgemeinen Formel

$$H - E'' - R_1 \qquad ,(VII)$$

in der

$R_1$ wie eingangs definiert ist und

E" ein Sauerstoffatom oder eine Sulfenylgruppe darstellt.

Die Umsetzung wird zweckmäßigerweise in der Weise durchgeführt, daß eine Verbindung der allgemeinen Formel VI, in einem geeigneten Lösungsmittel, z.B. in Wasser/Salzsäure, Methanol/Salzsäure oder Dioxan/Salzsäure, mit einem Nitrit, z.B. Natriumnitrit oder einem Ester der salpetrigen Säure, in ein Diazoniumsalz bei niederen Temperaturen, z.B. bei Temperaturen zwischen -10 und 5°C, übergeführt wird.

Das so erhaltene entsprechende Diazoniumsalz, z.B. das Hydrochlorid oder das Hydrosulfat, wird anschließend mit einer entsprechenden Verbindung der allgemeinen Formel VII gegebenenfalls in Gegenwart eines Reaktionsbeschleunigers wie Kupfer zweckmäßigerweise bei Temperaturen zwischen 0 und 50°C umgesetzt. Besonders vorteilhaft wird die Umsetzung jedoch in der Weise durchgeführt, daß eine wässrige Lösung des erhaltenen Diazoniumsalzes in eine Verbindung der allgemeinen Formel VII bzw. in eine vorgelegte Lösung einer Verbindung der allgemeinen Formel VII, z.B. in eine wässrige neutrale oder schwach basische Lösung, bei Raumtemperatur zugetropft wird.

Erhält man eine Verbindung der allgemeinen Formel I, in der $R_1$ eine Cyanoalkylgruppe darstellt, so kann diese anschließend mittels Alkoholyse und/oder Hydrolyse in eine entsprechende Verbindung der allgemeinen Formel I, in der $R_1$ eine Alkoxycarbonylalkylgruppe oder Carboxyalkylgruppe darstellt, übergeführt werden und/oder

eine Verbindung der allgemeinen Formel I, in der $R_1$ eine Carboxyalkylgruppe darstellt, mittels Veresterung in eine entsprechende Verbindung der allgemeinen Formel I, in der $R_1$ eine Alkoxycarbonylalkylgruppe darstellt, übergeführt werden.

Die nachträgliche Alkoholyse und/oder Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittels wie Wasser, Wasser/Methanol, Ethanol, Wasser/Ethanol, Wasser/-Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Die nachträgliche Veresterung wird zweckmäßigerweise in einem geeigneten Lösungsmittel, z.B. in einem entsprechenden Alkohol, Pyridin, Toluol, Methylenchlorid, Tetrahydrofuran oder Dioxan, in Gegenwart eines säureaktivierenden und/oder wasserentziehenden Mittels wie Thionylchlorid, Chlorameisensäureäthylester, N,N'-Carbonyldiimidazol oder N,N'-Dicyclohexylcarbodiimid oder dessen Isoharnstoffethern, gegebenenfalls in Gegenwart eines Reaktionsbeschleunigers wie Kupfer-chlorid, oder durch Umesterung, z.B. mit einem entsprechenden Kohlensäurediester, bei Temperaturen zwischen 0 und 100°C, vorzugsweise jedoch bei Temperaturen zwischen 20°C und der Siedetemperatur des betreffenden Lösungsmittels, durchgeführt.

Ferner können die erhaltenen Verbindungen der allgemeinen Formel I anschließend gewünschtenfalls in ihre physiologisch verträglichen Säureadditionssalze mit anorganischen oder organischen Säuren übergeführt werden. Als Säuren kommen hierfür beispielsweise Salzsäure, Bromwasserstoffsäure, Schwefelsäure, Phosphorsäure, Furmarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formeln II bis VII sind teilweise literaturbekannt bzw. erhält man nach literaturbekannten Verfahren.

So erhält man beispielsweise die als Ausgangsstoffe verwendeten Verbindungen der allgemeinen Formel II durch Acylierung von 4,5-Diamino-pyrimidin und die Verbindungen der allgemeinen Formeln III, IV und VI durch anschließende Kondensation mit einem entsprechenden Benzoesäurederivat und gegebenenfalls anschließende Oxidation bzw. Reduktion der 4-Nitrogruppe (siehe EP-A-0.022.495).

Wie bereits eingangs erwähnt weisen die neuen Verbindungen der allgemeinen Formel I, deren 1H Tautomere und deren physiologisch verträgliche Säureadditionssalze bei einer langen Wirkungsdauer überlegene pharmakologische Eigenschaften auf, insbesondere eine positiv inotrope Wirkung und/oder eine Wirkung auf den Blutdruck.

Beispielsweise wurden die Verbindungen

A = 8-(2-Methoxy-4-propargyloxyphenyl)-purin,

B = 8-(2-Methoxy-4-methoxycarbonylmethoxyphenyl)-purin und

C = 8-(2-Methoxy-4-cyanmethoxyphenyl)-purin

auf ihre biologischen Eigenschaften wie folgt untersucht:

1.) Bestimmung der Blutdruckwirkung und der positiv inotropen Wirkung an der narkotisierten Katze

Die Untersuchungen wurden an Katzen durchgeführt, die mit Pentobarbital-Natrium (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparamter $dp/dt_{max}$ mittels eines Analogdifferenzierers gewonnen. Die zu untersuchenden Substanzen wurden in eine Vena femoralis injiziert. Als Lösungsmittel diente physiologische Kochsalz-Lösung oder Polydiol 200. Jede Substanz wurde an mindestens 3 Katzen geprüft, Dosis 2 mg/kg i.v..

Die nachfolgende Tabelle enthält die Mittelwerte:

| Substanz | Dosis mg/kg i.v. | Zunahme von $dp/dt_{max}$ in % | Blutdrucksenkung in mm Hg |
|---|---|---|---|
| A | 2,0 | + 131 | - 42/- 41 |
| B | 2,0 | + 31 | + 15/+ 11 |
| C | 2,0 | + 97 | - 51/- 35 |

2. Akute Toxizität:

Die akute Toxizität der zu untersuchenden Substanzen wurde orientierend an Gruppen von je 10 Mäusen nach oraler Gabe einer Einzeldosis von 300 mg/kg bestimmt (Beobachtungszeit: 14 Tage):

| Substanz | Orientierend akute Toxizität |
|---|---|
| A | > 300 mg/kg (0 von 10 Tieren gestorben) |
| B | > 300 mg/kg (0 von 10 Tieren gestorben) |
| C | > 300 mg/kg (0 von 10 Tieren gestorben) |

Die neuen Verbindungen sind gut verträglich, so konnte bei der Untersuchung der Substanzen A bis C keinerlei herztoxische Wirkungen bzw. Kreislaufschäden beobachtet werden.

Aufgrund ihrer pharmakologischen Eigenschaften eignen sich die erfindungsgemäß hergestellten Verbindungen der allgemeinen Formel I sowie deren physiologisch verträgliche Säureadditionssalze zur Behandlung von Herzinsuffizienzen unterschiedlicher Genese, da sie die Kontraktionskraft des Herzens steigern und zum Teil durch die Blutdrucksenkung die Entleerung des Herzens erleichtern.

Hierzu lassen sich die neuen Verbindungen sowie deren physiologisch verträgliche Säureadditionssalze, gegebenenfalls in Kombination mit anderen Wirksubstanzen, in die üblichen pharmazeutischen Anwendungsformen wie Tabletten, Dragées, Pulver, Suppositorien, Suspensionen, Ampullen oder Tropfen einarbeiten. Die Einzeldosis beträgt hierbei 1-4 x täglich 0,3 - 2,2 mg/kg Körpergewicht, vorzugsweise jedoch 0,7 - 1,5 mg/kg Körpergewicht.

Die nachfolgenden Beispiele sollen die Erfindung näher erläutern:

Beispiel 1

8-(2-Methoxy-4-propargyloxyphenyl)-purin

2,06 g (10 mMol) 2-Methoxy-4-propargyloxybenzoesäure und 1,1 g (10 mMol) 4,5-Diamino-pyrimidin werden in 50 ml Phosphoroxychlorid 1 Stunde lang unter Rückfluß erhitzt. Das Phosphoroxychlorid wird daraufhin im Vakuum abdestilliert, der Rückstand in ca. 250 ml Wasser suspendiert, unter Rühren mit konz. Ammoniak neutralisiert und das Produkt daraufhin abgesaugt und durch Säulenchromatographie über Aluminiumoxyd (neutral, Aktivitätsstufe II) gereinigt (Elutionsmittel: Methylenchlorid mit 1 % Ethanol).

Ausbeute: 71,5 % der Theorie

Schmelzpunkt: 195-196°C

$C_{15}H_{12}N_4O_2$ (280,3)

| | | | | | | |
|---|---|---|---|---|---|---|
| Ber.: | C | 64,28 | H | 4,32 | N | 19,99 |
| Gef.: | | 64,36 | | 4,46 | | 20,04 |

Analog werden folgende Verbindungen erhalten:

8-(2-Methoxy-3-propargyloxyphenyl)-purin
8-(2-Methoxy-5-propargyloxyphenyl)-purin
8-(2-Methoxy-3-allyloxyphenyl)-purin
8-(2-Methoxy-4-allyloxyphenyl)-purin
8-(2-Methoxy-5-allyloxyphenyl)-purin
8-(2-Methoxy-3-propargylaminophenyl)-purin
8-(2-Methoxy-4-propargylaminophenyl)-purin
8-(2-Methoxy-5-propargylaminophenyl)-purin
8-(2-Methoxy-3-cyanmethylaminophenyl)-purin
8-(2-Methoxy-4-cyanmethylaminophenyl)-purin
8-(2-Methoxy-5-cyanmethylaminophenyl)-purin
8-(2-Methoxy-3-allylaminophenyl)-purin

8-(2-Methoxy-4-allylaminophenyl)-purin
8-(2-Methoxy-5-allylaminophenyl)-purin
8-(2-Ethoxy-4-propargyloxyphenyl)-purin
8-(2-Ethoxy-4-allyloxyphenyl)-purin
8-(2,4-Dipropargyloxyphenyl)-purin
8-(2,4-Dicyanmethoxyphenyl)-purin
8-(2,4-Diallyloxyphenyl)-purin
8-(2-Methoxy-4-buten-(2)-yloxyphenyl)-purin
8-(2-Methoxy-4-buten-(3)-yloxyphenyl)-purin
8-(2-Methoxy-4-penten-(2)-yloxyphenyl)-purin
8-(2-Methoxy-4-penten-(3)-yloxyphenyl)-purin
8-(2-Methoxy-4-buten-(2)-ylaminophenyl)-purin
8-(2-Methoxy-4-buten-(3)-ylaminophenyl)-purin
8-(2-Methoxy-4-penten-(2)-ylaminophenyl)-purin
8-(2-Methoxy-4-penten-(3)-ylaminophenyl)-purin
8-(2-Methoxy-4-butin-(2)-yloxyphenyl)-purin
8-(2-Methoxy-4-butin-(3)-yloxyphenyl)-purin
8-(2-Methoxy-4-pentin-(2)-yloxyphenyl)-purin
8-(2-Methoxy-4-pentin-(3)-yloxyphenyl)-purin
8-(2-Methoxy-4-butin-(2)-ylaminophenyl)-purin
8-(2-Methoxy-4-butin-(3)-ylaminophenyl)-purin
8-(2-Methoxy-4-pentin-(2)-ylaminophenyl)-purin
8-(2-Methoxy-4-pentin-(3)-ylaminophenyl)-purin
8-(2-Methoxy-4-α-cyanethoxyphenyl)-purin
8-(2-Methoxy-4-ß-cyanethoxyphenyl)-purin
8-(2-Methoxy-4-α-cyanethylaminophenyl)-purin
8-(2-Methoxy-4-ß-cyanethylaminophenyl)-purin

## Beispiel 2

8-(2-Methoxy-4-methoxycarbonylmethoxyphenyl)-purin

2,54 g (10 mMol) 2-Methoxy-4-methoxycarbonylmethoxy-benzoesäuremethylester und 1,1 g (10 mMol) 4,5-Diaminopyrimidin werden in einem Gemisch aus 80 ml Phosphoroxychlorid und 20

ml Sulfolan 2 Stunden lang unter Rückfluß erhitzt. Daraufhin wird das Phosphoroxychlorid im Vakuum abdestilliert, der Rückstand in 250 ml Wasser gegeben, unter Rühren mit konz. Ammoniak auf pH 8-9 eingestellt, dann die Lösung mit Natriumchlorid gesättigt und dreimal mit je 30 ml Methylethylketon extrahiert. Die organischen Extrakte werden im Vakuum eingeengt, das so erhaltene Rohprodukt durch Säulenchromatographie über Kieselgel gereinigt (Elutionsmittel: Methylenchlorid mit 1-6 % Ethanol).

Ausbeute: 11,1 % der Theorie,

Schmelzpunkt: 233-235°C

$C_{15}H_{14}N_4O_4$ (314,3)

| | | | | | | |
|---|---|---|---|---|---|---|
| Ber.: | C | 57,32 | H | 4,49 | N | 17,83 |
| Gef.: | | 57,00 | | 4,72 | | 17,36 |

Analog werden folgende Verbindungen erhalten:

8-(2-Methoxy-3-methoxycarbonylmethoxyphenyl)-purin
8-(2-Methoxy-5-methoxycarbonylmethoxyphenyl)-purin
8-(2-Methoxy-4-ethoxycarbonylmethylthiophenyl)-purin
8-(2-Methoxy-4-n-propyloxycarbonylmethylaminophenyl)-purin
8-(2-Ethoxy-4-methoxycarbonylmethoxyphenyl)-purin
8-(2-Methoxy-4-methoxycarbonylmethylthiophenyl)-purin
8-(2-Methoxy-4-methoxycarbonylmethylaminophenyl)-purin
8-(2-Methoxy-4-α-methoxycarbonylethoxyphenyl)-purin
8-(2-Methoxy-4-ß-methoxycarbonylethoxyphenyl)-purin
8-(2-Methoxy-4-α-methoxycarbonylethylthiophenyl)-purin
8-(2-Methoxy-4-ß-methoxycarbonylethylthiophenyl)-purin
8-(2-Methoxy-4-α-methoxycarbonylethylaminophenyl)-purin
8-(2-Methoxy-4-ß-methoxycarbonylethylaminophenyl)-purin

Beispiel 3

8-(2-Methoxy-4-cyanmethoxyphenyl)-purin

2,45 g (10 mMol) 8-(2-Methoxy-4-hydroxyphenyl)-purin werden

in 50 ml Dimethylformamid suspendiert und unter Rühren bei Raumtemperatur 1,23 g (11 mMol) Kalium-tert.butylat hinzugefügt. Die nun klare Lösung wird noch 30 Minuten lang gerührt, bevor 3 ml Chloracetonitril zugetropft werden. Die Reaktionslösung wird noch weitere 5 Stunden lang bei Raumtemperatur gerührt, dann auf 250 ml gesättigte Natriumchloridlösung gegossen. Das dabei ausgefallene Rohprodukt wird abgesaugt und durch Chromatographie über Kieselgel gereinigt (Elutionsmittel: Methylenchlorid mit 3-8 % Ethanol).

Ausbeute: 8,8 % der Theorie,
Schmelzpunkt: 240-242°C

| | | | | | | |
|---|---|---|---|---|---|---|
| $C_{14}H_{11}N_5O_2$ | | (281,3) | | | | |
| Ber.: | C | 59,78 | H | 3,94 | N | 24,90 |
| Gef.: | | 58,96 | | 4,13 | | 24,39 |

Analog werden folgende Verbindungen erhalten:

8-(2-Methoxy-3-cyanmethoxyphenyl)-purin
8-(2-Methoxy-5-cyanmethoxyphenyl)-purin
8-(2-Methoxy-3-cyanmethylthiophenyl)-purin
8-(2-Methoxy-4-cyanmethylthiophenyl)-purin
8-(2-Methoxy-5-cyanmethylthiophenyl)-purin
8-(2-Ethoxy-4-cyanmethoxyphenyl)-purin
8-(2-Methoxy-4-allylaminophenyl)-purin

## Beispiel 4

### 8-(2-Methoxy-4-allylthiophenyl)-purin

Zu einer Lösung von 1,2 g (5 mMol) 8-(2-Methoxy-4-aminophenyl)-purin in 15 ml halbkonzentrierter Schwefelsäure wird bei 0-5°C unter Rühren eine Lösung von 350 mg (5 mMol) Natriumnitrit in 2 ml Wasser gegeben. Unter Eiskühlung wird weitere 15 Minuten gerührt und diese Lösung dann bei 0-5°C innerhalb von 10 Minuten zu einer Mischung aus 0,5 ml Allylmercaptan, 400 mg Natriumhydroxid, 500 mg Kupferpulver und 30 ml Wasser getropft. Nach 2 Stunden Rühren bei 0-5°C wird

das Reaktionsgemisch filtriert und das Filtrat in 120 ml Eiswasser eingerührt, mit konzentrierter Ammoniaklösung neutralisiert und der ausgefallene Feststoff abgesaugt. Das so erhaltene Rohprodukt wird durch Säulenchromatographie über Kieselgel gereinigt (Elutionsmittel: Dichlormethan mit 5 % Ethanol). Das Produkt fällt amorph an.

Ausbeute: 8,8 % der Theorie

$C_{15}H_{14}N_4OS$ (298,4)

| | | | | | | | |
|---|---|---|---|---|---|---|---|
| Ber.: | C | 60,38 | H | 4,73 | N | 18,78 |
| Gef.: | | 60,66 | | 4,85 | | 17,99 |

Dünnschichtchromatographie (Kieselgel, Dichlormethan mit 10 % Ethanol): Rf = 0,6

Analog werden folgende Verbindungen erhalten:

8-(2-Methoxy-3-propargylthiophenyl)-purin
8-(2-Methoxy-5-propargylthiophenyl)-purin
8-(2-Methoxy-4-propargylthiophenyl)-purin
8-(2-Methoy-3-allylthiophenyl)-purin
8-(2-Methoxy-5-allylthiophenyl)-purin
8-(2-Dimethylamino-4-propargylthiophenyl)-purin
8-(2-Dimethylamino-4-allylthiophenyl)-purin
8-(2-Methoxy-4-buten-(2)-ylthiophenyl)-purin
8-(2-Methoxy-4-buten-(3)-ylthiophenyl)-purin
8-(2-Methoxy-4-butin-(2)-ylthiophenyl)-purin
8-(2-Methoxy-4-butin-(3)-ylthiophenyl)-purin

## Beispiel 5

### 8-(2-Dimethylamino-4-allyloxyphenyl)-purin

Zu einer Lösung von 1,0 g (3,88 mMol) 8-(2-Dimethylamino-4-aminophenyl)-purin in 15 ml halbkonzentrierter Schwefelsäure wird bei 0-5°C unter Rühren eine Lösung von 270 mg (4 mMol) Natriumnitrit in 3 ml Wasser zugetropft und anschließend

weitere 10 Minuten lang gerührt. Die so erhaltene Diazoniumsalz-Lösung wird dann unter Eiskühlung (0-5°C) tropfenweise in 15 ml Allylalkohol eingerührt, das Reaktionsgemisch bei Raumtemperatur 1 Stunde lang nachgerührt, dann in 150 ml Wasser gegossen und mit konzentrierter Ammoniaklösung alkalisch gestellt (pH 8). Nun wird dreimal mit je 30 ml Essigester extrahiert, die vereinigten Extrakte über wasserfreiem Natriumsulfat getrocknet, filtriert und im Vakuum eingeengt. Der feste Rückstand wird chromatographisch gereinigt (150 g Kieselgel, Elutionsmittel: Dichlormethan mit 1-3 % Ethanol). Das Produkt fällt amorph an.

Ausbeute: 5,2 % der Theorie

$C_{16}H_{17}N_5O$ (295,3)

| | | | | | | |
|---|---|---|---|---|---|---|
| Ber.: | C | 65,06 | H | 5,80 | N | 23,72 |
| Gef.: | | 65,13 | | 5,88 | | 23,70 |

$^1$H-NMR-Spektrum ($CDCl_3$ + $CD_3OD$):

$\delta$ = 2,83 (s,6H); 4,73 (breites d,2H); 5,5 (m,2H); 6,0 (m,1H); 6,9 (m,1H); 7,0 (d,1H); 8,4 (d,1H); 8,95 (s,1H); 9,04 (s,1H) ppm.

Analog werden folgende Verbindungen erhalten:

8-(2-Dimethylamino-4-propargyloxyphenyl)-purin

8-(2-Diethylamino-4-propargyloxyphenyl)-purin

Beispiel 6

8-(2-Dimethylamino-4-cyanmethoxyphenyl)-purin

Zu einer Lösung von 1,0 g (3,88 mMol) 8-(2-Dimethylamino-4-aminophenyl)-purin in 15 ml halbkonzentrierter Schwefelsäure wird bei 0-5°C unter Rühren eine Lösung von 270 mg (4 mMol) Natriumnitrit in 3 ml Wasser zugetropft und anschließend weitere 10 Minuten lang gerührt. Die so erhaltene Diazoniumsalzlösung wird dann unter Eiskühlung (0-5°C) tropfenweise in 15 ml Glycolsäurenitril eingerührt, das Reaktionsgemisch

bei Raumtemperatur 1 Stunde lang nachgerührt, dann in 150 ml Wasser gegossen und mit konzentrierter Ammoniaklösung alkalisch gestellt (pH 8). Nun wird dreimal mit je 20 ml Dichlormethan extrahiert, die vereinigten Extrakte über wasserfreiem Natriumsulfat getrocknet, filtriert und im Vakuum eingeengt. Der Rückstand wird chromatographisch gereinigt (150 g Aluminiumoxid neutral, Aktivitätsstufe II; Elutionsmittel: Dichlormethan mit 1-2 % Ethanol). Das Produkt fällt als Öl an.

Ausbeute: 10,4 % der Theorie,

$C_{15}H_{14}N_6O$ (294,3)

| | | | | | | |
|---|---|---|---|---|---|---|
| Ber.: | C | 61,21 | H | 4,79 | N | 28,56 |
| Gef.: | | 61,03 | | 4,71 | | 28,22 |

$^1$H-NMR-Spektrum: ($CDCl_3$):

$\delta$ = 2,80 (s,6H); 4,88 (s,2H); 6,91 (m,1H); 6,99 (d,1H); 8,51 (d,1H); 8,95 (s,1H); 9,06 (s,1H); 12,40 (breites s,1H) ppm.

Analog wird folgende Verbindung erhalten:

8-(2-Diethylamino-4-cyanmethoxyphenyl)-purin.

## Beispiel 7

8-(2,4-Dipropargyloxyphenyl)-purin

1,46 g (10,0 mMol) 4,5-Diamino-pyrimidin-hydrochlorid und 2,4 g (10,0 mMol) 2,4-Dipropargyloxybenzoesäure werden in 100 ml Phosphoroxychlorid unter Rühren eine Stunde lang zum Rückfluß erhitzt. Unter Kühlung wird das Phosphoroxychlorid anschließend mit Wasser zersetzt, das ausgefallene Rohprodukt abgesaugt, mit Wasser nachgewaschen und dann in 100 ml Wasser suspendiert. Unter Rühren wird die Suspension dann mit 5%iger Natriumhydrogencarbonat-Lösung auf pH 8-9 eingestellt und weitere dreißig Minuten lang gerührt. Dann wird

der Feststoff erneut abgesaugt und im Umlufttrockenschrank bei 60°C getrocknet. Durch Säulenchromatographie wird das so erhaltene Produkt gereinigt (150 g Kieselgel, Elutionsmittel: Dichlormethan mit 5 % Ethanol).
Ausbeute: 46,3 % der Theorie,
Schmelzpunkt: 217-219°C
$C_{17}H_{12}N_4O_2$ (304,3)

| | | | | | | |
|---|---|---|---|---|---|---|
| Ber.: | C | 67,10 | H | 3,98 | N | 18,41 |
| Gef.: | | 67,11 | | 4,07 | | 18,41 |

Analog werden folgende Verbindungen erhalten:

8-(2,3-Dipropargyloxyphenyl)-purin,
8-(2,5-Dipropargyloxyphenyl)-purin,
8-(3,4-Dipropargyloxyphenyl)-purin,
8-(3,5-Dipropargyloxyphenyl)-purin
8-(2,4-Diallyloxyphenyl)-purin
8-(2,5-Diallyloxyphenyl)-purin
8-(3,4-Diallyloxyphenyl)-purin
8-(3,5-Diallyloxyphenyl)-purin
8-(2,4-Dicyanomethoxyphenyl)-purin
8-(2,5-Dicyanomethoxyphenyl)-purin
8-(3,4-Dicyanomethoxyphenyl)-purin
8-(3,5-Dicyanomethoxyphenyl)-purin

## Beispiel 8

8-(2-Methoxy-4-propargylsulfinylphenyl)-purin und
8-(2-Methoxy-4-propargylsulfonylphenyl)-purin

---

730 mg (2,47 mMol) 8-(2-Methoxy-4-propargylthiophenyl)-purin werden in 10 ml Eisessig gelöst, dann 2 ml Wasserstoffperoxid (30 %) hinzugegeben und bei Raumtemperatur gerührt. Nach fünf Stunden wird dünnschichtchromatographisch Umsetzung zu einem Produktgemisch festgestellt, in dem auch noch

Ausgangsmaterial enthalten ist. Das Reaktionsgemisch wird daraufhin auf 100 ml Wasser gegossen, mit konzentriertem Ammoniak schwach alkalisch gestellt, mit Natriumchlorid gesättigt und fünfmal mit je 10 ml Dichlormethan extrahiert. Die vereinigten Extrakte werden über wasserfreiem Magnesiumsulfat getrocknet und dann zur Trockne eingeengt. Aus dem so erhaltenen Produktgemisch werden dann säulenchromatographisch die beiden S-oxidierten Produkte gewonnen (120 g Kieselgel, Aktivitätsstufe I, Elutionsmittel: Dichlormethan mit 5-20 % Ethanol).

Ausbeute:

a) 170 mg (22 % der Theorie) Sulfoxid (amorph)
$R_f$-Wert: 0,18 (Kieselgel, Laufmittel: Methylenchlorid/Ethanol = 9:1)

b) 234 mg (29 % der Theorie) Sulfon (amorph)
$R_f$-Wert: 0,38 (Kieselgel, Laufmittel: Methylenchlorid/Ethanol = 9:1)

## Beispiel A

Tabletten zu 100 mg 8-(2-Methoxy-4-propargyloxyphenyl)-purin

Zusammensetzung
1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Feuchtsiebung: 1,5 mm
Trocknen: Umlufttrockenschrank 50°C
Trockensiebung: 1 mm
Dem Granulat die restlichen Hilfsstoffe zumischen und Endmischung zu Tabletten verpressen.
Tablettengewicht: 175 mg
Stempel: 8 mm

## Beispiel B

Dragées zu 50 mg 8-(2-Methoxy-4-propargyloxyphenyl)-purin

Zusammensetzung:
1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

| | |
|---|---|
| Feuchtsiebung: | 1,0 mm |
| Trockensiebung: | 1,0 mm, |
| Trocknung: | 50°C im Umlufttrockenschrank |

Granulat und restliche Hilfsstoffe mischen und zu Kernen verpressen.

| | |
|---|---|
| Kerngewicht: | 80 mg |
| Stempel: | 6 mm |
| Wölbungsradius: | 5 mm |

Die fertigen Kerne werden auf übliche Weise mit einem Zuckerüberzug im Dragierkessel versehen.

Drageegewicht: 120 mg

## Beispiel C

**Suppositorien zu 75 mg 8-(2-Methoxy-4-propargyloxyphenyl)-purin**

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

### Herstellungsverfahren:

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

Beispiel D

Ampullen zu 50 mg 8-(2-Methoxy-4-propargyloxyphenyl)-purin

1 Ampulle enthält:

| | |
|---|---|
| Wirksubstanz | 50,0 mg |
| Sorbit | 250,0 mg |
| Dest. Wasser ad | 5,0 ml |

Herstellungsverfahren:

Die Wirksubstanz und Sorbit werden in dest. Wasser gelöst, dann wird das angegebene Volumen aufgefüllt und steril filtriert.

Abfüllung: in Ampullen zu 5 ml
Sterilisation: 20 Minuten bei 120°C

Beispiel E

Tropfen zu 250 mg 8-(2-Methoxy-4-propargyloxyphenyl)-purin

| | | | |
|---|---|---|---|
| Wirksubstanz | | 5,0 | g |
| p-Oxybenzoesäuremethylester | | 0,035 | g |
| p-Oxybenzoesäurepropylester | | 0,015 | g |
| Anisöl | | 0,05 | g |
| Menthol | | 0,06 | g |
| Saccharin-Natrium | | 1,0 | g |
| Glycerin | | 10,0 | g |
| Ethanol | | 40,0 | g |
| Dest. Wasser | ad | 100,0 | ml |

Herstellungsverfahren:

Die Benzoesäureester werden in Ethanol gelöst und anschließend das Anisöl und das Menthol zugegeben. Dann wird die Wirksubstanz, Glycerin und Saccharin-Natrium im Wasser gelöst zugegeben. Die Lösung wird anschließend klar filtriert.

Patentansprüche

1. Purinderivate der allgemeinen Formel

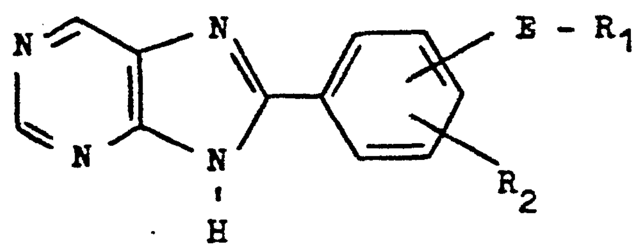

(I)

in der

E ein Sauerstoff- oder Schwefelatom, eine Imino-, Sulfinyl- oder Sulfonylgruppe,

$R_1$ eine Alkylgruppe mit 1 bis 3 Kohlenstoffatomen, welche durch eine Cyan-, Carboxy- oder Alkoxycarbonylgruppe mit insgesamt 2 bis 6 Kohlenstoffatomen substituiert ist, eine Alkenyl- oder Alkinylgruppe mit jeweils 3 bis 5 Kohlenstoffatomen und

$R_2$ eine Alkoxy-, Cyanoalkoxy-, Alkylamino- oder Dialkylaminogruppe, wobei der Alkylteil jeweils 1 bis 3 Kohlenstoffatome enthalten kann, eine Alkenyloxy- oder Alkinyloxygruppe mit jeweils 3 bis 5 Kohlenstoffatomen bedeuten, deren Tautomere und deren Säureadditionssalze.

2. Purinderivate gemäß Anspruch 1, in der

E ein Sauerstoffatom, eine Imino-, Sulfenyl-, Sulfinyl- oder Sulfonylgruppe,

$R_1$ eine Allyl-, Propargyl-, Cyanomethyl- oder Alkoxycarbonylmethylgruppe, in der der Alkoxyteil 1 bis 3 Kohlenstoffatome enthalten kann, und

$R_2$ eine Methoxy-, Ethoxy-, Allyloxy-, Propargyloxy-, Cyanomethoxy-, oder Dimethylaminogruppe bedeuten, deren Tautomere und deren Säureadditionssalze.

3. Purinderivate gemäß Anspruch 1, in der E, $R_1$ und $R_2$ wie im Anspruch 2 definiert sind, der Rest $R_1$-E- in 4-Stellung und der Rest $R_2$ in 2-Stellung des Phenylkerns steht, deren Tautomere und deren Säureadditionssalze.

4. 8-(2-Methoxy-4-propargyloxyphenyl)-purin, dessen Tautomere und Säureadditionssalze.

5. 8-(2-Methoxy-4-methoxycarbonylmethoxy-phenyl)-purin, dessen Tautomere und Säureadditionssalze.

6. 8-(2-Methoxy-4-cyanmethoxyphenyl)-purin, dessen Tautomere und Säureadditionssalze.

7. Physiologisch verträgliche Säureadditionssalze der Verbindungen gemäß den Ansprüchen 1 bis 6 mit anorganischen oder organischen Säuren.

8. Arzneimittel, enthaltend eine Verbindung gemäß den Ansprüchen 1 bis 6 oder ein physiologisch verträgliches Säureadditionssalz gemäß Anspruch 7 neben einem oder mehreren inerten Trägerstoffen und/oder Verdünnungsmitteln.

9. Verfahren zur Herstellung eines Arzneimittels gemäß Anspruch 8, dadurch gekennzeichnet, daß auf nichtchemischem Wege eine Verbindung gemäß den Ansprüchen 1 bis 6 oder ein physiologisch verträgliches Säureadditionssalz gemäß Anspruch 7 in einem oder mehreren inerten üblichen Trägerstoffen und/oder Verdünnungsmittelsn eingearbeitet wird.

10. Verfahren zur Herstellung der Verbindungen gemäß den Ansprüchen 1 bis 7, dadurch gekennzeichnet, daß

 0142075

a) eine gegebenenfalls im Reaktionsgemisch gebildete Verbindung der allgemeinen Formel

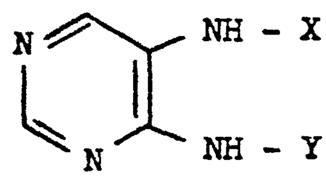

(II)

in der
einer der Reste X oder Y ein Wasserstoffatom und der andere der beiden Reste X und Y oder beide Reste X und Y eine Gruppe der Formel

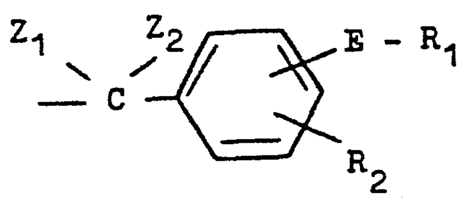

darstellen, in der

$R_1$, $R_2$ und E wie eingangs definiert sind,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder

b) zur Herstellung einer Verbindung der allgemeinen Formel I, in der E eine Sulfinyl- oder Sulfonylgruppe darstellt, eine Verbindung der allgemeinen Formel

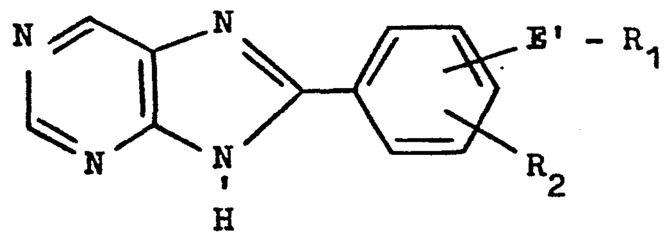

(III)

in der

$R_1$ und $R_2$ wie eingangs definiert sind und

E' eine Sulfenyl- oder Sulfinylgruppe darstellt, oxidiert wird oder

c) zur Herstellung einer Verbindung der allgemeinen Formel I, in der E eine Iminogruppe, ein Sauerstoff- oder Schwefelatom darstellt, eine Verbindung der allgemeinen Formel

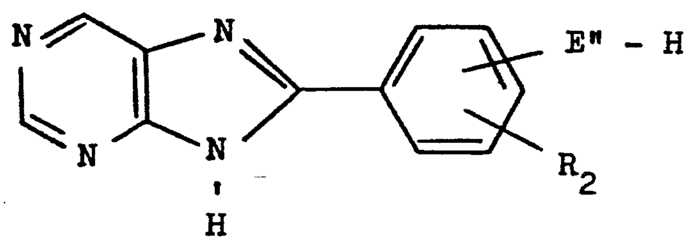

(IV)

in der

$R_2$ wie eingangs definiert ist und

E" eine Iminogruppe, ein Sauerstoff- oder Schwefelatom darstellt, mit einer Verbindung der allgemeinen Formel

$$R_1 - V \qquad ,(V)$$

in der

$R_1$ wie eingangs definiert ist und

V eine nukleophile Austrittsgruppe darstellt, umgesetzt wird oder

d) zur Herstellung einer Verbindung der allgemeinen Formel I, in der E ein Sauerstoff- oder Schwefelatom dar- stellt, eine Verbindung der allgemeinen Formel

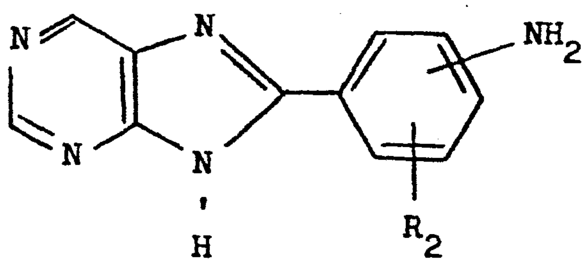

(VI)

in der

$R_2$ wie eingangs definiert ist, mit einem Nitrit und anschließend das so erhaltene Diazoniumsalz mit einer Verbindung der allgemeinen Formel

$$H - E"' - R_1 \quad ,(VII)$$

in der

$R_1$ wie eingangs definiert ist und

E" ein Sauerstoff- oder Schwefelatom darstellt, umgesetzt wird

und gewünschtenfalls anschließend eine so erhaltene Verbindung der allgemeinen Formel I, in der $R_1$ eine Cyanoalkylgruppe darstellt, mittels Alkoholyse und/oder Hydrolyse in eine entsprechende Verbindung der allgemeinen Formel I, in der $R_1$ eine Alkoxycarbonylalkyl- oder Carboxyalkylgruppe darstellt, übergeführt wird oder

eine so erhaltene Verbindung der allgemeinen Formel I, in der $R_1$ eine Carboxyalkylgruppe darstellt, mittels Veresterung in eine entsprechende Verbindung der allgemeinen Formel I, in der $R_1$ eine Alkoxycarbonylalkylgruppe darstellt, übergeführt wird oder

eine so erhaltene Verbindung der allgemeinen Formel I in ihr Säureadditionssalz, insbesondere in ihr physiologisch verträgliches Säureadditionssalz, mit einer anorganischen oder organischen Säure übergeführt wird.

Patentansprüche (Bennungsland: AT)

1. Verfahren zur Herstellung von neuen Purinderivaten der allgemeinen Formel

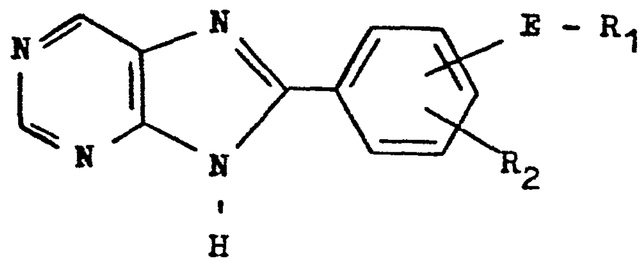

(I)

in der

E ein Sauerstoff- oder Schwefelatom, eine Imino-, Sulfinyl- oder Sulfonylgruppe,

$R_1$ eine Alkylgruppe mit 1 bis 3 Kohlenstoffatomen, welche durch eine Cyan-, Carboxy- oder Alkoxycarbonylgruppe mit insgesamt 2 bis 6 Kohlenstoffatomen substituiert ist, eine Alkenyl- oder Alkinylgruppe mit jeweils 3 bis 5 Kohlenstoffatomen und

$R_2$ eine Alkoxy-, Cyanoalkoxy-, Alkylamino- oder Dialkylaminogruppe, wobei der Alkylteil jeweils 1 bis 3 Kohlenstoffatome enthalten kann, eine Alkenyloxy- oder Alkinyloxygruppe mit jeweils 3 bis 5 Kohlenstoffatomen bedeuten, von deren Tautomeren und von deren Säureadditionssalzen, dadurch gekennzeichnet, daß

a) eine gegebenenfalls im Reaktionsgemisch gebildete Verbindung der allgemeinen Formel

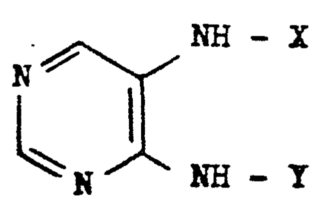

(II)

in der

einer der Reste X oder Y ein Wasserstoffatom und der andere der beiden Reste X und Y oder beide Reste X und Y eine Gruppe der Formel

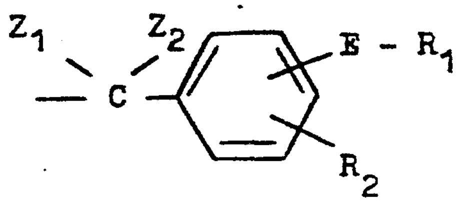

darstellen, in der

$R_1$, $R_2$ und E wie eingangs definiert sind,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder

b) zur Herstellung einer Verbindung der allgemeinen Formel I, in der E eine Sulfinyl- oder Sulfonylgruppe darstellt, eine Verbindung der allgemeinen Formel

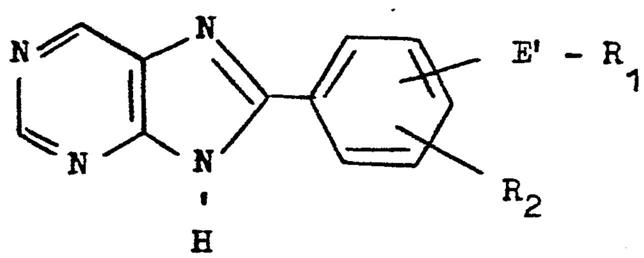

,(III)

in der
$R_1$ und $R_2$ wie eingangs definiert sind und
E' eine Sulfenyl- oder Sulfinylgruppe darstellt, oxidiert wird oder

c) zur Herstellung einer Verbindung der allgemeinen Formel I, in der E eine Iminogruppe, ein Sauerstoff- oder Schwefelatom darstellt, eine Verbindung der allgemeinen Formel

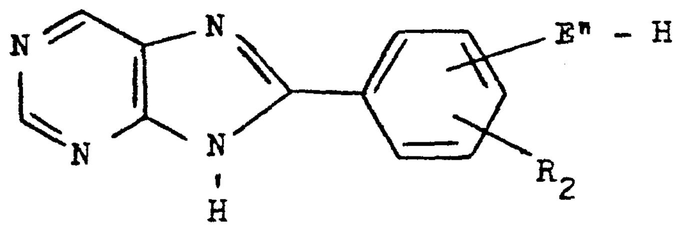

(IV)

in der
$R_2$ wie eingangs definiert ist und
E" eine Iminogruppe, ein Sauerstoff- oder Schwefelatom darstellt, mit einer Verbindung der allgemeinen Formel

$$R_1 - V \quad ,(V)$$

in der
$R_1$ wie eingangs definiert ist und
V eine nukleophile Austrittsgruppe darstellt, umgesetzt wird oder

d) zur Herstellung einer Verbindung der allgemeinen Formel I, in der E ein Sauerstoff- oder Schwefelatom darstellt, eine Verbindung der allgemeinen Formel

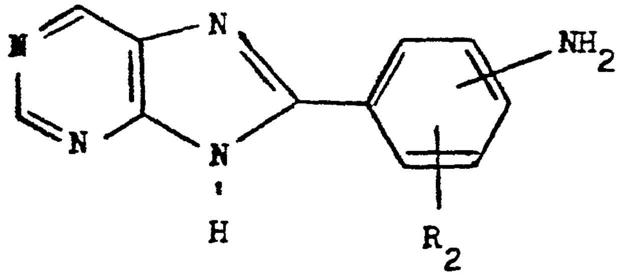

(VI)

in der
$R_2$ wie eingangs definiert ist, mit einem Nitrit und anschließend das so erhaltene Diazoniumsalz mit einer Verbindung der allgemeinen Formel

$$H - E''' - R_1 \qquad ,(VII)$$

in der
$R_1$ wie eingangs definiert ist und
$E'''$ ein Sauerstoff- oder Schwefelatom darstellt, umgesetzt wird

und gewünschtenfalls anschließend eine so erhaltene Verbindung der allgemeinen Formel I, in der $R_1$ eine Cyanoalkylgruppe darstellt, mittels Alkoholyse und/oder Hydrolyse in eine entsprechende Verbindung der allgemeinen Formel I, in der $R_1$ eine Alkoxycarbonylalkyl- oder Carboxyalkylgruppe darstellt, übergeführt wird oder

eine so erhaltene Verbindung der allgemeinen Formel I, in der $R_1$ eine Carboxyalkylgruppe darstellt, mittels Veresterung in eine entsprechende Verbindung der allgemeinen Formel I, in der $R_1$ eine Alkoxycarbonylalkylgruppe darstellt, übergeführt wird oder

eine so erhaltene Verbindung der allgemeinen Formel I in ihr Säureadditionssalz, insbesondere in ihr physiologisch verträgliches Säureadditionssalz, mit einer anorganischen oder organischen Säure übergeführt wird.

2. Verfahren zur Herstellung der Verbindungen gemäß Anspruch 1, dadurch gekennzeichnet, daß

a) eine gegebenenfalls im Reaktionsgemisch gebildete Verbindung der allgemeinen Formel

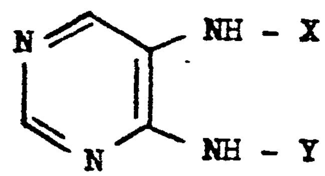

(II)

in der

einer der Reste X oder Y ein Wasserstoffatom und der andere der beiden Reste X und Y oder beide Reste X und Y eine Gruppe der Formel

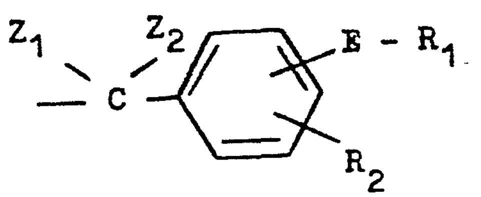

darstellen, in der

$R_1$, $R_2$ und E wie eingangs definiert sind,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder

b) zur Herstellung einer Verbindung der allgemeinen Formel I, in der E eine Sulfinyl- oder Sulfonylgruppe darstellt, eine Verbindung der allgemeinen Formel

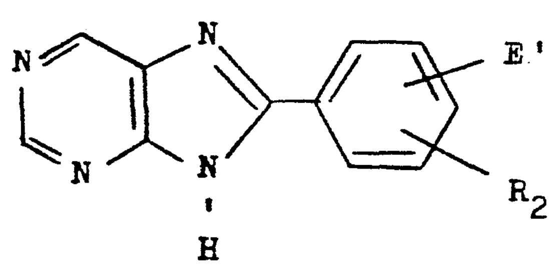

,(III)

in der
$R_1$ und $R_2$ wie eingangs definiert sind und
E' eine Sulfenyl- oder Sulfinylgruppe darstellt, oxidiert wird oder

c) zur Herstellung einer Verbindung der allgemeinen Formel I, in der E ein Sauerstoffatom oder eine Sulfenylgruppe darstellt, eine Verbindung der allgemeinen Formel

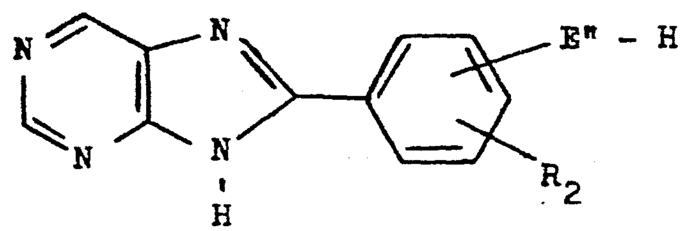

(IV)

in der
$R_2$ wie eingangs definiert ist und
E" ein Sauerstoffatom oder eine Sulfenylgruppe darstellt, mit einer Verbindung der allgemeinen Formel

$$R_1 - V \qquad ,(V)$$

in der
$R_1$ wie eingangs definiert ist und
V eine nukleophile Austrittsgruppe darstellt, umgesetzt wird oder

d) zur Herstellung einer Verbindung der allgemeinen Formel I, in der E ein Sauerstoffatom oder eine Sulfenylgruppe darstellt, eine Verbindung der allgemeinen Formel

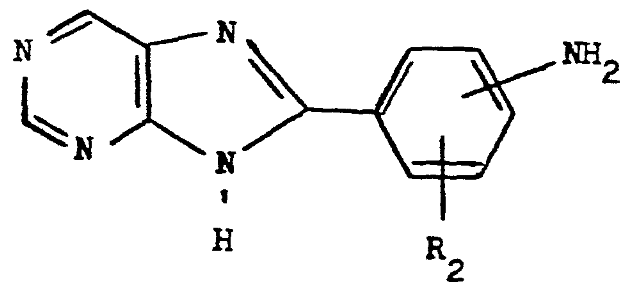

(VI)

in der $R_2$ wie eingangs definiert ist, mit einem Nitrit und anschließend das so erhaltene Diazoniumsalz mit einer Verbindung der allgemeinen Formel

$$H - E'' - R_1 \qquad ,(VII)$$

in der $R_1$ wie eingangs definiert ist und E" ein Sauerstoffatom oder eine Sulfenylgruppe darstellt, umgesetzt wird

und gewünschtenfalls anschließend eine so erhaltene Verbindung der allgemeinen Formel I, in der $R_1$ eine Cyanoalkylgruppe darstellt, mittels Alkoholyse und/oder Hydrolyse in eine entsprechende Verbindung der allgemeinen Formel I, in der $R_1$ eine Alkoxycarbonylalkyl- oder Carboxyalkylgruppe darstellt, übergeführt wird oder

eine so erhaltene Verbindung der allgemeinen Formel I, in der $R_1$ eine Carboxyalkylgruppe darstellt, mittels Veresterung in eine entsprechende Verbindung der allgemeinen Formel I, in der $R_1$ eine Alkoxycarbonylalkylgruppe darstellt, übergeführt wird oder

eine so erhaltene Verbindung der allgemeinen Formel I in ihr Säureadditionssalz, insbesondere in ihr physiologisch verträgliches Säureadditionssalz, mit einer anorganischen oder organischen Säure übergeführt wird.

3. Verfahren gemäß den Ansprüchen 1 und 2, dadurch gekennzeichnet, daß die Umsetzung in einem Lösungsmittel durchgeführt wird.

4. Verfahren gemäß den Ansprüchen 1a, 2a und 3, dadurch gekennzeichnet, daß die Umsetzung in Gegenwart eines Kondensationsmittels, eines Acylierungsmittels oder einer Base durchgeführt wird.

5. Verfahren gemäß den Ansprüchen 1a, 2a, 3 und 4, dadurch gekennzeichnet, daß die Umsetzung bei Temperaturen zwischen 0 und 250°C, vorzugsweise jedoch bei der Siedetemperatur des Reaktionsgemisches, durchgeführt wird.

6. Verfahren gemäß den Ansprüchen 1b, 2b und 3, dadurch gekennzeichnet, daß zur Herstellung einer Sulfinylverbindung der allgemeinen Formel I die Oxidation mit einem Äquivalent des verwendeten Oxidationsmittels durchgeführt wird.

7. Verfahren gemäß den Ansprüchen 1b, 2b, 3 und 6, dadurch gekennzeichnet, daß die Oxidation bei Temperaturen zwischen -80 und 100°C, vorzugsweise jedoch bei Temperaturen zwischen 0 und 50°C, durchgeführt wird.

8. Verfahren gemäß den Ansprüchen 1c, 2c und 3, dadurch gekennzeichnet, daß die Umsetzung in Gegenwart eines säurebindenden Mittels durchgeführt wird.

9. Verfahren gemäß den Ansprüchen 1c, 2c, 3 und 8, dadurch gekennzeichnet, daß die Umsetzung bei Temperaturen zwischen 0 und 100°C, vorzugsweise bei Temperaturen zwischen Raumtemperatur und 50°C, durchgeführt wird.

10. Verfahren gemäß den Ansprüchen 1d, 2d und 3, dadurch gekennzeichnet, daß die Umsetzung des Diazoniumsalzes bei Temperaturen zwischen 0 und 50°C, vorzugsweise bei Raumtemperatur, durchgeführt wird.